# EUROPEAN PATENT APPLICATION

(11) **EP 2 638 861 A1**
(43) Date of publication of application: **18.09.2013**
(21) Application number: 13158727.1
(22) Date of filing: 12.03.2013
(51) Int. Cl.: A61B 8/00

(54) **Probe for ultrasonic diagnostic apparatus**

(30) Priority: 13.03.2012 KR 20120025504
(71) Applicant: Samsung Medison Co., Ltd., Gangwon-do 250-870 (KR)
(72) Inventor: Kim, Ji Seon, Daegu (KR); Kim, Mi-Ri, Gangnma-gu Seoul (KR); Park, Jung-Lim, Gyeonggi-do (KR); Seo, Min Seon, Gyeongsangbuk-do (KR)
(74) Representative: Lorenz, Markus

(57) **Abstract**

A probe for an ultrasonic diagnostic apparatus includes a transducer module that transmits and receives ultrasonic waves. The transducer module includes a piezoelectric device transmitting and receiving ultrasonic waves, at least one matching layer disposed on the front surface of the piezoelectric device, a backing layer disposed on the rear surface of the piezoelectric device, a backing block disposed on the rear surface of the backing layer, and a gas layer disposed between the backing layer and the backing block. Acoustic energy proceeding in the backward direction of the piezoelectric device is reflected by the gas layer toward the piezoelectric device.

## Description

### BACKGROUND

### 1. Field

Embodiments of the present disclosure relate to a probe for an ultrasonic diagnostic apparatus that transmits ultrasonic waves to an object to be diagnosed and receives ultrasonic waves reflected by the object.

### 2. Description of the Related Art

In general, an ultrasonic diagnostic apparatus is an apparatus that emits ultrasonic waves through the surface of an object to an internal body region of the object to be diagnosed, and acquires tomograms of soft tissues or images of blood flow via the reflected ultrasonic waves.

The ultrasonic diagnostic apparatus includes a probe that transmits an ultrasonic signal to the object and receives a signal reflected by the object while remaining in contact with the object.

The probe includes a transducer module transmitting and receiving ultrasonic waves as described above. The transducer module includes a piezoelectric device transmitting and receiving ultrasonic waves, a matching layer disposed on the front surface of the piezoelectric device and reducing an acoustic impedance difference between the object and the piezoelectric device, and a backing layer and a backing block sequentially disposed on the rear surface of the piezoelectric device and absorbing ultrasonic waves proceeding in the backward direction of the piezoelectric device.

### SUMMARY

It is an aspect of the present disclosure to provide a probe for an ultrasonic diagnostic apparatus having a transducer module with improved sensitivity.

Additional aspects of the present disclosure will be set forth in part in the description which follows and, in part, will be obvious from the description, or may be learned by practice of the disclosed subject matter.

In accordance with one aspect of the present disclosure, a probe for an ultrasonic diagnostic apparatus includes a transducer module to transmit and receive ultrasonic waves. The transducer module includes a piezoelectric device transmitting and receiving ultrasonic waves, at least one matching layer disposed on the front surface of the piezoelectric device, a backing layer disposed on the rear surface of the piezoelectric device, a backing block disposed on the rear surface of the backing layer, and a gas layer disposed between the backing layer and the backing block.

In some embodiments, the gas layer may contain air.

In some embodiments, an acoustic impedance of the backing layer may be greater than an acoustic impedance of the backing block.

In some embodiments, at least one of the rear surface of the backing layer and the front surface of the backing block may include a gas layer-forming groove to form the gas layer.

In some embodiments, the gas layer-forming groove may include a first gas layer-forming groove disposed at the rear surface of the backing layer.

In some embodiments, the first gas layer-forming groove may have a flat inner surface.

In some embodiments, the first gas layer-forming groove may have a curved inner surface having a depth gradually decreasing from the center to both ends thereof.

In some embodiments, the gas layer-forming groove may include a second gas layer-forming groove disposed at the front surface of the backing block.

In some embodiments, the second gas layer-forming groove may have a flat inner surface.

In some embodiments, the second gas layer-forming groove may have a curved inner surface having a depth gradually decreasing from the center to both ends thereof.

In some embodiments, the gas layer has a thickness of λ/16 to λ/2, where λ is a wavelength of the ultrasonic waves.

In some embodiments, a thickness of the backing layer and the backing block is in the range of λ/8 to λ/2, where λ is a wavelength of the ultrasonic waves.

### BRIEF DESCRIPTION OF THE DRAWINGS

These and/or other aspects of the disclosure will become apparent and more readily appreciated from the following description of the embodiments, taken in conjunction with the accompanying drawings as follows:
FIG. 1 is an exemplary perspective view illustrating a probe for an ultrasonic diagnostic apparatus according to an embodiment of the present disclosure;
FIG. 2 is a n exemplary cross-sectional view illustrating a probe for an ultrasonic diagnostic apparatus according to an embodiment of the present disclosure; and
FIGS. 3 to 7 are exemplary cross-sectional views respectively illustrating probes for an ultrasonic diagnostic apparatus according to other embodiments of the present disclosure.

### DETAILED DESCRIPTION

Reference will now be made in detail to the embodiments of the present disclosure, examples of which are illustrated in the accompanying drawings, wherein like reference numerals refer to like elements throughout.

Hereinafter, a probe 10 for an ultrasonic diagnostic apparatus according to an embodiment of the present disclosure will be described in detail with reference to the drawings.

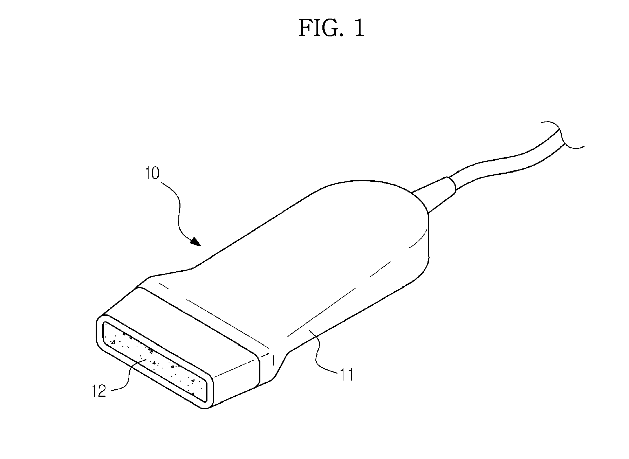

As illustrated in FIG. 1, the probe 10 for an ultrasonic diagnostic apparatus according to the present embodiment includes a housing 11 defining an appearance of the probe 10 and a probe lens 12 disposed at the front end of the housing 11, which is to contact a body region of an object to be diagnosed.

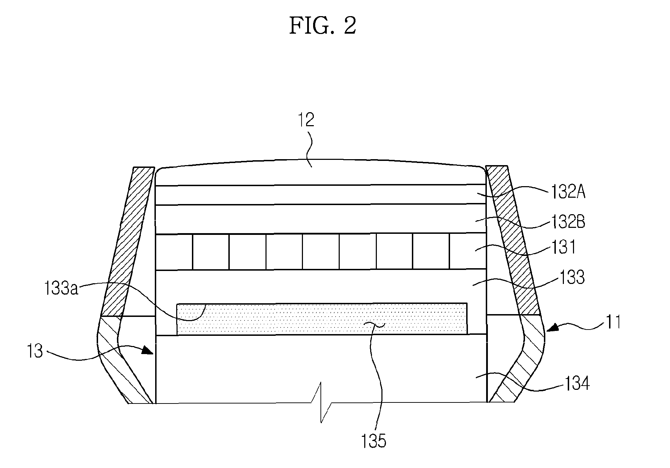

As shown in FIG. 2, a transducer module 13 to transmit and receive ultrasonic waves is disposed behind the probe lens 12 in the housing 11.

The transducer module 13 includes a piezoelectric device 131 that transmits ultrasonic waves to the object to be diagnosed and receives ultrasonic waves reflected by the object, matching layers 132A and 132B disposed on the front surface of the piezoelectric device 131, and a backing layer 133 and a backing block 134 sequentially disposed on the rear surface of the piezoelectric device 131.

The piezoelectric device 131 converts electrical energy applied thereto into ultrasonic waves and transmits the ultrasonic waves in the forward direction, and/or receives ultrasonic waves reflected by the object and converts the ultrasonic waves into electrical energy.

The matching layers 132A and 132B are disposed between the piezoelectric device 131 and the object, and reduce an acoustic impedance difference between the piezoelectric device 131 and the object. According to the present embodiment, the matching layers 132A and 132B include a first matching layer 132A and a second matching layer 132B having different acoustic impedances. When a plurality of matching layers 132A and 132B having different acoustic impedances are sequentially aligned as described above, the acoustic impedance difference may be reduced in a stepwise manner.

The backing layer 133 and the backing block 134 are respectively formed of materials absorbing ultrasound. Acoustic impedances of the backing layer 133 and the backing block 134 may be the same or may be combined in various ways to obtain a desired acoustic impedance such that, for example, the acoustic impedance of one of the backing layer 133 and the backing block 134 is greater than that of the other. According to the present embodiment, a thickness of the backing layer 133 and the backing block 134 is within a range of λ/8 to λ/2, where λ is a wavelength of the ultrasonic waves.

In addition, a gas layer 135 filled with a gas is disposed between the backing layer 133 and the backing block 134. According to the present embodiment, the gas layer 135 is filled with air and a first gas layer-forming groove 133a is formed at the rear surface of the backing layer 133 to form the gas layer 135. According to the present embodiment, the first gas layer-forming groove 133a has a flat inner surface, and a thickness of the first gas layer-forming groove 133a is in the range of λ/16 to λ/2, where λ is the wavelength of the ultrasonic waves.

By forming the gas layer 135 between the backing layer 133 and the backing block 134 as described above, acoustic energy proceeding in the backward direction of the piezoelectric device 131 is reflected by the interface between the piezoelectric device 131 and the gas layer 135 toward the piezoelectric device 131 due to acoustic impedance difference between the backing layer 133 and the gas layer 135, and received by the piezoelectric device 131. As a result, sensitivity of the transducer module 13 is improved.

As a result of experiments in which acoustic impedances of the backing layer 133 and the backing block 134 were varied in the transducer module 13 including the gas layer, it was confirmed that sensitivity of the transducer module 13 is improved when the backing layer 133 has a relatively greater acoustic impedance than the backing block 134.

According to the present embodiment, the gas layer 135 is filled with air. However, the disclosure is not limited thereto, and any material that is a gas at a room temperature may be used in the gas layer 135.

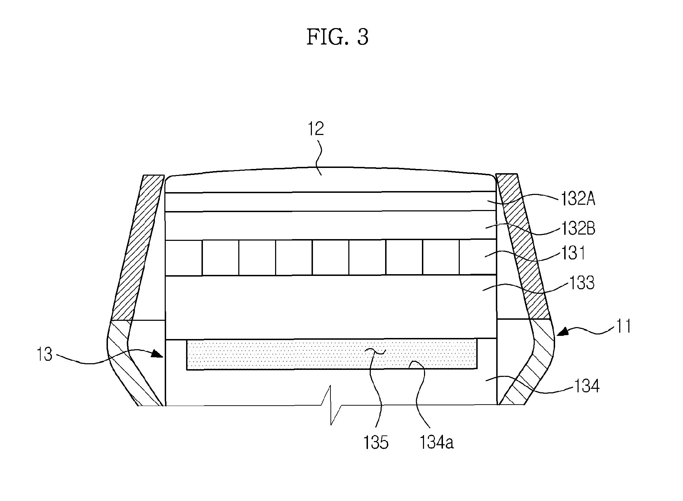

In addition, the gas layer 135 having a flat inner surface is formed at the rear surface of the backing layer 133 by the first gas layer-forming groove 133a according to the present embodiment. However, the disclosure is not limited thereto, and various modifications may be made as shown in FIGS. 3 to 7.

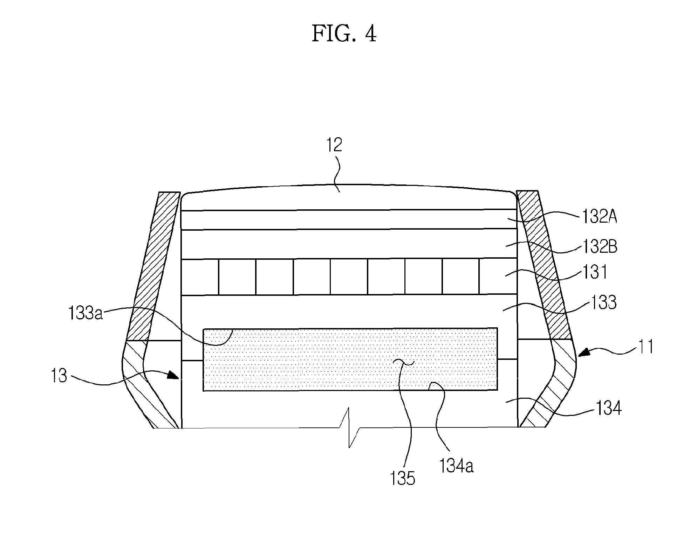

Referring to FIG. 3, the backing layer 133 does not have an element used to form the gas layer 135. The gas layer 135 is formed by a second gas layer-forming groove 134a disposed at the front surface of the backing block 134. Referring to FIG. 4, the transducer module 13 includes a first gas layer-forming groove 133a disposed at the rear surface of the backing layer 133 and a second gas layer-forming groove 134a disposed at the front surface of the backing block 134 to correspond to the first gas layer-forming groove 133a.

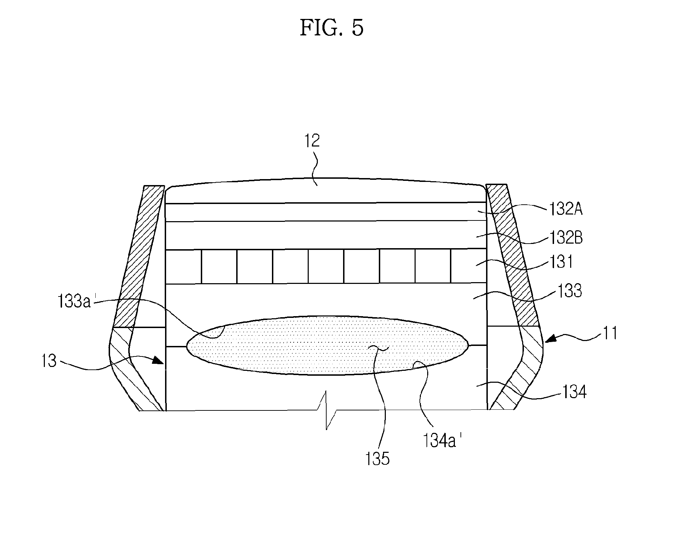

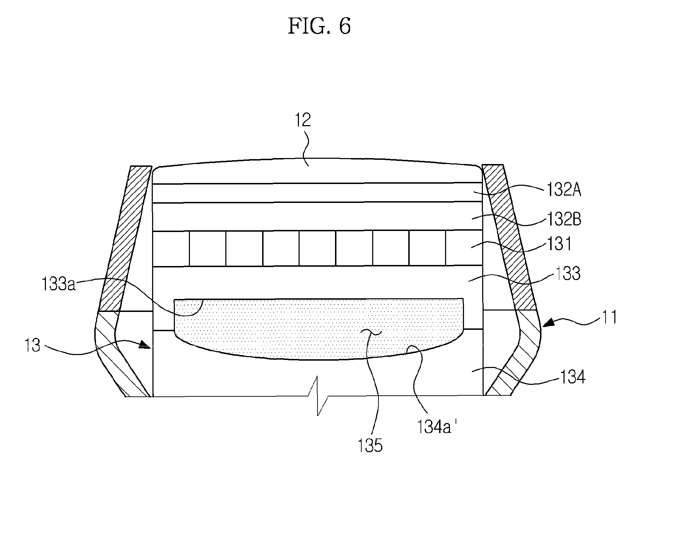

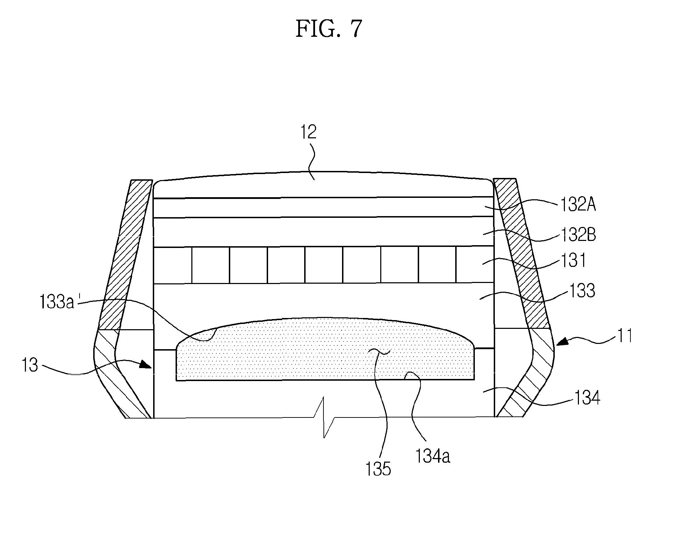

According to the foregoing embodiments, the first gas layer-forming groove 133a or the second gas layer-forming groove 134a have flat inner surfaces. However, the disclosure is not limited thereto. As illustrated in FIG. 5, a first gas layer-forming groove 133a' and a second gas layer-forming groove 134a' may be formed to have curved inner surfaces such that depths of the gas layer 135 gradually decrease from the center to both ends thereof. As illustrated in FIG. 6, the first gas layer-forming groove 133a may have a flat inner surface, and the second gas layer-forming groove 134a' may have a curved inner surface. As illustrated in FIG. 7, the first gas layer-forming groove 133a' may have a curved inner surface, and the second gas layer-forming groove 134a may have a flat inner surface, and various modifications may also be made.

As is apparent from the above description, the probe for an ultrasonic diagnostic apparatus reflects acoustic energy proceeding in the backward direction of the piezoelectric device toward the piezoelectric device by the gas layer disposed between the backing layer and the backing block. Thus, sensitivity of the transducer module is improved.

Although some embodiments of the present disclosure have been shown and described, it would be appreciated by those skilled in the art that changes may be made in these embodiments without departing from the principles and spirit of the subject matter of the present disclosure, the scope of which is defined in the claims and their equivalents.

## Claims

1. A probe for an ultrasonic diagnostic apparatus, the probe comprising a transducer module to transmit and receive ultrasonic waves,
wherein the transducer module comprises:
a piezoelectric device transmitting and receiving the ultrasonic waves;
at least one matching layer disposed on a front surface of the piezoelectric device;
a backing layer disposed on a rear surface of the piezoelectric device;
a backing block disposed on a rear surface of the backing layer; and
a gas layer disposed between the backing layer and the backing block.

2. The probe according to claim 1, wherein the gas layer comprises air.

3. The probe according to claim 1, wherein an acoustic impedance of the backing layer is greater than an acoustic impedance of the backing block.

4. The probe according to claim 1, wherein at least one of the rear surface of the backing layer and a front surface of the backing block comprises a gas layer-forming groove to form the gas layer.

5. The probe according to claim 4, wherein the gas layer-forming groove comprises a first gas layer-forming groove disposed at the rear surface of the backing layer.

6. The probe according to claim 5, wherein the first gas layer-forming groove has a flat inner surface.

7. The probe according to claim 5, wherein the first gas layer-forming groove has a curved inner surface having a depth gradually decreasing from a center to both ends thereof.

8. The probe according to claim 4, wherein the gas layer-forming groove comprises a second gas layer-forming groove disposed at the front surface of the backing block.

9. The probe according to claim 8, wherein the second gas layer-forming groove has a flat inner surface.

10. The probe according to claim 8, wherein the second gas layer-forming groove has a curved inner surface having a depth gradually decreasing from a center to both ends thereof.

11. The probe according to claim 1, wherein the gas layer has a thickness of λ/16 to λ/2, where λ is a wavelength of the ultrasonic waves.

12. The probe according to claim 1, wherein a thickness of the backing layer and the backing block is in the range of λ/8 to λ/2, where λ is a wavelength of the ultrasonic waves.

13. The probe according to claim 5, wherein the gas layer-forming groove comprises a second gas layer-forming groove disposed at the front surface of the backing block.

14. The probe according to claim 13, wherein the second gas layer-forming groove has a flat inner surface.

15. The probe according to claim 13, wherein the second gas layer-forming groove has a curved inner surface having a depth gradually decreasing from a center to both ends thereof.
